# EUROPEAN PATENT APPLICATION

(11) **EP 4 116 321 A1**
(43) Date of publication of application: **11.01.2023**
(21) Application number: 21787965.9
(22) Date of filing: 15.04.2021
(51) Int. Cl.: C07K 16/08, G01N 33/569, C12P 21/08

(54) **ADENOVIRUS IMMUNIZATION MEASUREMENT METHOD AND IMMUNIZATION MEASUREMENT DEVICE**

(30) Priority: 16.04.2020 JP 2020073233
(71) Applicant: Denka Company Limited, Tokyo 103-8338 (JP)
(72) Inventor: MIYAZAWA, Takashi, Gosen-shi, Niigata 959-1834 (JP); KUWAHARA, Miwa, Gosen-shi, Niigata 959-1834 (JP)
(74) Representative: Mewburn Ellis LLP
(86) International application number: PCT/JP2021/015538
(87) International publication number: WO 2021/210630

(57) **Abstract**

An object of the present invention is to provide: a monoclonal antibody that makes it possible that adenovirus contained in a test specimen is detected and measured rapidly, simply, and with high-sensitivity; and an immunoassay for adenovirus and an immunoassay device therefor, for both of which the monoclonal antibody is used. The present invention provides: a monoclonal antibody or an antigen-binding fragment thereof, which undergoes antigen-antibody reaction with a hexon trimer of adenovirus; and an immunoassay and an immunoassay device that are used with the monoclonal antibody.

## Description

### TECHNICAL FIELD

The present invention relates to an immunoassay for adenovirus and an immunoassay device therefor, and to an anti-adenovirus antibody for the immunoassay and the device.

### BACKGROUND ART

Adenovirus is known as a pathogen of the following: a respiratory disease such as acute febrile pharyngitis, pharyngoconjunctivitis, acute airway inflammation, or viral pneumonia; an eye disease such as acute follicular conjunctivitis or epidemic keratoconjunctivitis; a gastrointestinal disease such as transmissible gastroenteritis; a urologic disease such as urethritis; or the like. At present, adenovirus is classified into seven species A to G, and there are more than 80 types of adenovirus. It has been reported that the types up to type 51 are serotypes, and that the types from type 52 and above are genotypes based on the determination of the entire base sequence (Non-patent Document 1). When humans are infected with adenovirus, the humans can exhibit various clinical symptoms, and do not often exhibit a specific pathological condition, and thus, it is difficult to prove adenovirus infection from a clinical symptom. In addition, adenovirus is very infectious, and preventing herd infection is considered to involve proving viral infection early.

Examples of methods developed to detect adenovirus rapidly and simply include an immunochromatography to be performed with an anti-adenovirus antibody and a method to be performed with EIA. However, in the ophthalmologic field where a specimen can be collected only in a small amount, the positive ratio is 60% or less, and there is a demand for a rapid higher-sensitivity diagnostic method or an anti-adenovirus monoclonal antibody usable for such a method.

The infectious disease surveillance of National Institute of Infectious Diseases provides the information that there are patients who have been found to have pharyngoconjunctival fever, transmissible gastroenteritis, or epidemic keratoconjunctivitis as an adenovirus-associated disease. It is known that acute respiratory disease and pharyngoconjunctival fever are caused by the adenovirus species B, C, and E, that transmissible gastroenteritis is caused by the adenovirus species A, F, and G, and that epidemic keratoconjunctivitis is caused by the adenovirus species B, D, and E (Non-Patent Document 2). The species B adenovirus type 3 and the species E adenovirus type 4 are the most common etiology of epidemic keratoconjunctivitis and pharyngoconjunctival fever, and the species D adenovirus type 8, type 19, and type 37 are also the causes of a serious outbreak of epidemic keratoconjunctivitis in some countries, particularly in East Asia and Southeast Asia. Adenovirus type 8, type 19, and type 37 are well known as the epidemiologic causes of nosocomial infection. Nosocomial infection induced by adenovirus has recently posed a notable social issue in public hygiene and an economical and ethical issue in hospitals.

Up to now, a plurality of monoclonal antibodies that react with adenovirus have been produced and reported. For example, disclosed is a method in which adenovirus is detected using a monoclonal antibody that reacts with a specific subtype of adenovirus (Patent Documents 1 and 2). In addition, an adenovirus detection kit to be used with a monoclonal antibody for adenovirus is commercially available.

### PRIOR ART DOCUMENTS

### [Non-patent Documents]

[Non-patent Document 1] Seto D, et al., J Virol 85: 5701-5702, 2011
[Non-patent Document 2] IASR Vol. 38, p.133-135: July 2017
[Patent Document 1] JP 2000-290298 A
[Patent Document 2] JP 2000-290299 A

### SUMMARY OF THE INVENTION

### PROBLEMS TO BE SOLVED BY THE INVENTION

However, none of the monoclonal antibodies for adenovirus that are currently produced have sufficient detection sensitivity for adenovirus, and there is a demand for a monoclonal antibody that reacts with higher sensitivity.

In addition, some commercially available adenovirus detection kits come with a manual stating that a monoclonal antibody for a hexone of an adenovirus is used for the kit. However, the structure of a hexon in a sample can change, depending on the composition of a liquid extract used for the detection kit, and thus, the epitope for the monoclonal antibody has not been necessarily clear.

An object of the present invention is to provide: a monoclonal antibody that makes it possible that adenovirus contained in a test specimen is detected and measured rapidly, simply, and with high-sensitivity; and an immunoassay for adenovirus and an immunoassay device therefor, for both of which the monoclonal antibody is used.

### MEANS FOR SOLVING THE PROBLEMS

As a result of intensive study on the above-mentioned problems, the present inventors have discovered that, in order to detect adenovirus with higher sensitivity, it is effective to use a monoclonal antibody whose epitope is a hexon trimer contained in adenovirus, thereby completing the present invention.

That is, the present invention is as follows.
[1] A monoclonal antibody or an antigen-binding fragment thereof, which undergoes antigen-antibody reaction with a hexon trimer of adenovirus.
[2] The monoclonal antibody or the antigen-binding fragment thereof according to [1], wherein the monoclonal antibody undergoes antigen-antibody reaction more strongly with the hexon trimer than with a hexon monomer.
[3] An immunoassay for adenovirus, including performing the immunoassay of adenovirus by using antigen-antibody reaction between the monoclonal antibody or the antigen-binding fragment thereof according to [1] or [2] and adenovirus in a sample.
[4] The immunoassay according to [3], wherein the immunoassay is a sandwich method, and the monoclonal antibody or the antigen-binding fragment thereof is used as at least any one of a label or a solid phase.
[5] An immunoassay device for adenovirus, including the monoclonal antibody or the antigen-binding fragment thereof according to claim [1] or [2].

### EFFECTS OF THE INVENTION

The present invention can provide: a monoclonal antibody that makes it possible that adenovirus contained in a test specimen is detected and measured rapidly, simply, and with high-sensitivity; and an immunoassay for adenovirus and an immunoassay device therefor, for both of which the monoclonal antibody is used.

### BRIEF DESCRIPTION OF THE DRAWINGS

Fig. 1 is a diagram illustrating the results of Western blotting performed in Example 3.
Fig. 2 is a diagram illustrating the results obtained by staining, with CBB, the gel electrophoresed in Example 3.

### MODE FOR CARRYING OUT THE INVENTION

Below, embodiments of the present invention will be described in detail.

### <Monoclonal Antibody or Antigen-binding Fragment thereof>

A monoclonal antibody or an antigen-binding fragment thereof according to the present invention undergoes antigen-antibody reaction with a hexon trimer constituting adenovirus, and the epitope is a hexon trimer of adenovirus. In addition, the monoclonal antibody or the antigen-binding fragment thereof according to the present invention does not undergo antigen-antibody reaction with a hexon monomer constituting adenovirus.

Adenovirus is constituted by a linear double-stranded DNA and a regular-icosahedral capsid protein covering the DNA. The capsid protein is constituted by penton capsomeres located at the tops and 240 hexons. The present inventors have discovered that a monoclonal antibody or an antigen-binding fragment thereof that undergoes antigen-antibody reaction more strongly with a hexon trimer than with a hexon monomer of adenovirus is effective to detect adenovirus with high sensitivity.

Performing an immunoassay of adenovirus using such a monoclonal antibody or an antigen-binding fragment thereof makes it possible to detect a wide range of adenovirus subtypes and subgroups (subspecies) with high sensitivity. In addition, a specific signal due to antigen-antibody reaction can be detected at 200 to 300 kD as the molecular weight of the hexon trimer when the antibody is used for detection of adenovirus by Western blotting.

A monoclonal antibody or an antigen-binding fragment thereof according to the present invention has a basic structure composed of a heavy chain and a light chain, and the heavy chain and the light chain have the respective variable regions that can specifically bind to an antigen. V_{H} refers to the variable region of the heavy chain, and V_{L} refers to the variable region of the light chain. The variable region of the heavy chain and that of the light chain each contain amino acid sequences of complementarity-determining regions (CDR), that is, CDR1, CDR2, and CDR3, and a framework region (FR). For example, the variable region contains three or four FRs (for example, FR1, FR2, FR3, and optionally FR4) together with the three CDRs.

Examples of a monoclonal antibody of the present invention include quadruple-chain antibodies (for example, having two light chains and two heavy chains), recombinant antibodies, or modified antibodies (for example, chimeric antibodies, humanized antibodies, human antibodies, CDR transplanted antibodies, primatized antibodies, deimmunized antibodies, synhumanized (synhumanized) antibodies, half antibodies, and bispecific antibodies). The class of the monoclonal antibody is not limited to IgG, and may also be IgM or IgY.

In the present invention, an antigen-binding fragment of a monoclonal antibody is a fragment that is an antigen-binding site alone separated from the monoclonal antibody. Examples of such a fragment include fragments having specific antigen-binding capacity, such as Fab, Fab', F(ab')2, and single-chain antibodies (scFv) prepared by known methods.

### (Method of Preparing Monoclonal Antibody)

The monoclonal antibody of the present invention may be obtained by immunizing an animal with a complex or an extract containing a hexon trimer of adenovirus or with the hexon trimer of adenovirus or a partial peptide thereof by a known immunological method, and then preparing a hybridoma using cells of the immunized animal. The length of the peptide used for the immunization is not limited. Preferably, a peptide of not less than 5 amino acids, more preferably not less than 10 amino acids, may be used to provide the immunogen.

The immunogen can be obtained from a culture liquid, or can be obtained by incorporating DNA encoding a hexon trimer of adenovirus into a plasmid vector, and introducing the resulting vector to a host cell, followed by allowing expression of the hexon trimer. Alternatively, the hexon trimer of adenovirus or the partial peptide thereof to be used as the immunogen can be expressed as a fusion protein with a protein exemplified below, and the expressed fusion protein can be used as the immunogen after purification or without purification. The preparation of the fusion protein can be carried out using, for example, Glutathion S-transferase (GST), maltose-binding protein (MBP), thioredoxin (TRX), Nus-tag, S-tag, HSV-tag, FRAG tag, polyhistidine tag, or the like which is commonly used as a "protein expression/purification tag" by those skilled in the art. Preferably, the fusion protein with such a protein is cleaved into the portion of the hexon trimer of adenovirus or the partial peptide thereof, and the tag portion, using a digestive enzyme, and subjected to separation/purification before use as the immunogen.

The preparation of the monoclonal antibody from the immunized animal can be easily carried out by the well-known method of Kohler *et al.* (Kohler et al., Nature, vol. 256, p. 495-497 (1975)). That is, antibody-producing cells such as spleen cells or lymphocytes are recovered from the immunized animal, and the recovered cells are fused with mouse myeloma cells by a conventional method to prepare hybridomas. The resulting hybridomas are cloned by the limiting dilution method or the like. Thereafter, a monoclonal antibody that undergoes antigen-antibody reaction with the antigen used for the immunization of the animal is selected from the monoclonal antibodies produced by the cloned hybridomas.

Purification of the monoclonal antibody from ascites or a culture supernatant may be carried out by a known immunoglobulin purification method. Examples of the method include fractionation by salting out using ammonium sulfate or sodium sulfate, PEG fractionation, ethanol fractionation, DEAE ion-exchange chromatography, and gel filtration. Depending on the species of the immunized animal and the class of the monoclonal antibody, affinity chromatography using a carrier to which any of protein A, protein G, and protein L is bound may be used for the purification.

### <Immunoassay>

In the present invention, using the above-mentioned monoclonal antibody or an antigen-binding fragment thereof makes it possible to detect a wide range of adenovirus subtypes and subgroups (subspecies) with very high sensitivity. In the following description preceding the Examples section, a "monoclonal antibody" means a "monoclonal antibody or an antigen-binding fragment thereof", unless otherwise obvious from the context.

In the present invention, adenovirus is detected by performing an immunoassay of adenovirus using antigen-antibody reaction between the above-mentioned monoclonal antibody and adenovirus in a sample. That a monoclonal antibody undergoes antigen-antibody reaction with adenovirus means that a monoclonal antibody reacts specifically with adenovirus. The term "specific" means that, in a liquid system containing a mixture of antigen proteins and the monoclonal antibody, the antibody does not cause antigen-antibody reaction with components other than the antigen proteins at a detectable level, or, even in cases where the antibody causes a certain binding reaction or association reaction with such a component, the reaction is evidently weaker than the antigen-antibody reaction between the antibody and the antigen proteins.

In the present invention, examples of immunoassays that can be used include any method well known to those skilled in the art, such as a competition method, condensation method, Western blotting, immunostaining method, sandwich method, and the like.

A sandwich method is preferable as an immunoassay in the present invention. The sandwich method per se is well known in the field of immunoassays, and can be carried out by, for example, immunochromatography or ELISA. These sandwich methods per se are well known, and the method of the present invention can be carried out in the same manner as a well-known sandwich method except that the above-mentioned monoclonal antibody of the present invention that undergoes antigen-antibody reaction with a hexon trimer is used.

In a sandwich method, two kinds of antibodies (an immobilized antibody immobilized in a solid phase and a labeled antibody) that recognize an antigen are used. In a method of the present invention, at least any one of these two kinds of antibodies is the above-mentioned monoclonal antibody of the present invention.

In the immunoassay based on the detection principle of a sandwich method, any solid phase may be used as the solid phase on which the antibody is to be immobilized, as long as the antibody can be immobilized thereon by a known technique. The solid phase may be arbitrarily selected from known solid phases such as porous thin films (membranes) having a capillary action; particles, test tubes, and resin plates. Examples of the substance for labeling the antibody include enzymes, radioisotopes, fluorescent substances, luminescent substances, colored particles, and colloidal particles. Among the above-mentioned immunoassay methods using various materials, lateral-flow immunoassay methods using a membrane are preferred from the viewpoint of enabling simple and rapid clinical tests.

In cases where adenovirus is quantified or semi-quantified using the monoclonal antibody in the present invention, such quantification or semi-quantification involves "measurement" inevitably, and thus, is included in the "measurement" in the present invention. That is, in the present invention, "measurement" in an immunoassay includes any of quantification, semi-quantification, and detection.

### EXAMPLES

The present invention is described below by way of Examples. However, the present invention is not limited to the following Examples.

### (Example 1: Preparation of Anti-adenovirus Monoclonal Antibody)

### 1. Preparation of Adenovirus Antigen

Adenovirus was allowed to infect mammalian cells sensitive thereto. The resulting cells were cultured for some days. Then, the culture liquid of the adenovirus-infected cells were inactivated by ultraviolet irradiation and made ready for use.

### 2. Preparation of Anti-adenovirus Monoclonal Antibody

BALB/c mice were immunized with the adenovirus-inactivated antigen prepared in 1, and kept for a certain period. From each mouse, the spleen was removed, and fusion with mouse myeloma cells (P3×63) was carried out by the method of Kohler *et al.* (Kohler et al., Nature, vol. 256, p. 495-497 (1975)) to obtain a plurality of hybridoma cell lines that produce anti-adenovirus antibodies.

The obtained cell line was intraperitoneally administered to pristane-treated BALB/c mice. About two weeks later, antibody-containing ascites was collected. From the ascites obtained, IgG was purified by affinity chromatography using a protein A column, to obtain a plurality of purified anti-adenovirus monoclonal antibodies.

In the below-mentioned Examples, two antibodies, an antibody 1 and an antibody 2, were used, which were selected from a plurality of the resulting anti-adenovirus monoclonal antibodies, considering reactivity and specificity.

### (Example 2; Immunoassay Device for Measuring Adenovirus)

### 1. Immobilization of Anti-adenovirus Antibody on Nitrocellulose Membrane

The anti-adenovirus antibody (antibody 2) prepared in Example 1 was diluted with a buffer. The resulting liquid and an anti-mouse IgG antibody were made ready for use. The anti-adenovirus antibody and the anti-mouse IgG antibody were linearly applied respectively to the sample pad side and the absorber side of a nitrocellulose membrane lined with a PET film. Then, the nitrocellulose membrane was dried sufficiently under warm air to obtain a membrane having an anti-adenovirus antibody immobilized thereon.

### 2. Immobilization of Anti-adenovirus Antibody on Colored Polystyrene Particles

The anti-adenovirus antibody (antibody 1) prepared in Example 1 was covalently bound to colored polystyrene particles, and the colored polystyrene particles were suspended in a suspension. Then, colored polystyrene particles to which the anti-adenovirus antibodies were bound and which were dispersed sufficiently by ultrasonication were obtained. In the present description, the particles obtained here are referred to as "anti-adenovirus antibody-immobilized particles".

### 3. Application/Drying of Colored Polystyrene Particles to Which Anti-adenovirus Antibody is Bound

A predetermined amount of the anti-adenovirus antibody-immobilized particles prepared in 2 were applied to a glass-fiber non-woven fabric, and the non-woven fabric was then dried sufficiently under warm air. In the present description, the pad obtained here is referred to as a "labeled antibody pad".

### 4. Production of Adenovirus Test Device

The anti-adenovirus antibody-immobilized membrane prepared in 1 and the labelled antibody pads prepared in 2 and 3 were laminated with other members (backing sheet, absorption zone, and sample pad), and the resulting laminate was cut into a piece with a width of 5 mm, to provide an adenovirus test device.

### 5. Confirmation of Reactivity of Adenovirus Test Device

A culture liquid of adenovirus-infected cells of each type was diluted with a buffer to prepare a two-fold dilution series of each type of adenovirus.

The adenovirus diluted liquid prepared was added to a sample suspension (10 mM Tris (pH 8.0), 1 w/v% polyoxyethylene octylphenyl ether, 3 w/v% arginine, and 3 w/v% BSA), and 50 µL of the resulting mixture was added dropwise to the adenovirus test device produced in 4. Then, the test device was left to stand for 5 minutes.

In cases where coloring could be visually observed at both the position where the anti-mouse IgG antibody was applied and the position where the anti-adenovirus antibody was applied, the test result was evaluated as + (positive). In cases where coloring could be visually observed only at the position where the anti-mouse IgG antibody was applied and where coloring could not be visually observed at the position where the anti-adenovirus antibody was applied, the test result was evaluated as - (negative). In cases where coloring could not be visually observed at the position where the anti-mouse IgG antibody was applied, the test result was evaluated as invalid.

The lowest adenovirus concentration at which the test result was evaluated as positive was regarded as the lowest detection sensitivity. The results are shown in Table 1.

**[Table 1]**

| Adenovirus | | Lowest Detection Sensitivity | |
|---|---|---|---|
| Type | Species | TCID₅₀/Test | copies/Test |
| 1 | C | 1.95×10² | 2.50×10³ |
| 2 | C | 1.95×10² | 6.25×10³ |
| 3 | B | 2.79×10² | 1.25×10⁴ |
| 5 | C | 7.85×10¹ | 6.25×10³ |
| 6 | C | 5.58×10² | 3.13×10³ |
| 7 | B | 1.74×10² | 1.25×10⁴ |
| 8 | D | 1.95×10¹ | 1.25×10³ |
| 11 | B | 2.79×10² | 5.00×10⁴ |
| 19 | D | 2.44×10¹ | 1.25×10⁴ |
| 31 | A | 4.48×10² | 1.25×10⁵ |
| 37 | D | 3.14×10¹ | 2.50×10³ |

As shown in Table 1, it was possible to confirm that the immunoassay device used with the anti-adenovirus antibody of the present invention reacted with many types of adenovirus belonging to the species A to D.

In addition it was possible to confirm that the device reacted with each of the subtypes: type 53, type 54, type 56, type 64, type 79, type 81, and type 85.

### 6. Confirmation of Specificity of Adenovirus Test Device

To the adenovirus test device produced in 4, 50 µL of a sample suspension containing virus that induces respiratory infection was added dropwise, and the test device was left to stand for 5 minutes.

In cases where coloring could be visually observed at both the position where the anti-mouse IgG antibody was applied and the position where the anti-adenovirus antibody was applied, the test result was evaluated as +. In cases where coloring could be visually observed only at the position where the anti-mouse IgG antibody was applied and where coloring could not be visually observed at the position where the anti-adenovirus antibody was applied, the test result was evaluated as -. In cases where coloring could not be visually observed at the position where the anti-mouse IgG antibody was applied, the test result was evaluated as invalid.

The results are shown in Table 2.

**[Table 2]**

| Virus | Measurement Results |
|---|---|
| Coxsackievirus type A9 | - |
| Coxsackievirus type B4 | - |
| Coxsackievirus type B5 | - |
| Coxsackievirus type B6 | - |
| Echo virus type 2 | - |
| Echo virus type 3 | - |
| Echo virus type 4 | - |
| Echo virus type 6 | - |
| Echo virus type 9 | - |
| Echo virus type 11 | - |
| Echo virus type 30 | - |
| Herpes simplex virus type 1 | - |
| Human Metapneumovirus type A | - |
| Human Metapneumovirus type B | - |
| Influenza virus A/New Caledonia/20/99 (H1N1) | - |
| Influenza virus A/Beijing/262/95 (H1N1) | - |
| Influenza virus A/New York/55/2004 (H3N2) | - |
| Influenza virus A/Hiroshima/52/2005 (H3N2) | - |
| Influenza virus B/Shanghai/361/2002 (Yamagata) | - |
| Influenza virus B/Malaysia/2506/2004 (Victoria) | - |
| Measles virus | - |
| Mumps virus | - |
| Parainfluenza virus type 1 | - |
| Parainfluenza virus type 2 | - |
| Parainfluenza virus type 3 | - |
| Parainfluenza virus type 4 | - |
| RS virus Long strain (type A) | - |
| RS virus CH-18 strain (type B) | - |

As shown in Table 2, it was possible to confirm that the adenovirus immunoassay device used with the anti-adenovirus antibody of the present invention reacted specifically with adenovirus, because the immunoassay device reacted with adenovirus, but exhibited no crossreactivity with the etiological virus of any other respiratory infection.

### 7. Performance Comparison of Adenovirus Test Device

The lowest detection sensitivity was compared between the adenovirus test device (the present device) prepared in 4 and a commercially available adenovirus kit.

Culture liquids of adenovirus-infected cells were each diluted with a buffer to prepare two-fold dilution series. To the present device, 50 µL of a sample suspension containing an adenovirus diluted liquid was added dropwise, and the present device was left to stand for 5 minutes and evaluated. The commercially available kit was used with a prescribed amount of adenovirus diluted liquid as a sample, and the test result was evaluated by performing a test in accordance with the document attached to each kit.

Assuming that the largest adenovirus dilution ratio at which the test result was evaluated as positive with the present device is "1", the largest dilution ratio at which the test result was evaluated as positive with a commercially available adenovirus kit was regarded as relative sensitivity, and is shown in Table 3.

**[Table 3]**

| Kit | Relative Sensitivity of Each Type (Species)^{*} | | | | | | |
|---|---|---|---|---|---|---|---|
| | Type 1 (C) | Type 2 (C) | Type 3 (B) | Type 4 (E) | Type 11 (B) | Type 37 (D) | Type 54 (D) |
| Present Kit | 1 | 1 | 1 | 1 | 1 | 1 | 1 |
| Kit A | 1 | 1/2 | 1 | 1 | 1 | 1 | 1 |
| Kit B | 1/2 | 1/4 | 1/2 | 1/2 | 1/2 | 1/2 | 1/4 |
| Kit C | 1/8 | 1/8 | 1/8 | 1/20 | 1/20 | NT | NT |
| Kit D | 1 | 1/2 | 1/2 | 1/2 | 1/2 | 1/2 | 1/2 |
| Kit E | 1/16 | 1/32 | 1/100 | 1/80 | 1/400 | NT | NT |
| Kit F | 1/2 | 1/4 | 1/2 | 1/2 | 1/2 | 1/2 | 1/4 |
| Kit G | 1/4 | 1/8 | 1/8 | 1/8 | 1/4 | 1/4 | 1/4 |
| Kit H | 1/8 | 1/4 | 1/4 | 1/4 | 1/4 | 1/4 | 1/4 |

| | | | | | | | |
|---|---|---|---|---|---|---|---|
| NT: Not tested | | | | | | | |

As shown in Table 3, it was possible to confirm that the immunoassay device used with the anti-adenovirus antibody of the present invention had the highest reactivity with adenovirus type 2, and also had the highest reactivity with another type of adenovirus in the same manner as the kit A did.

### (Example 3: Antigen Recognition Site of Anti-adenovirus Monoclonal Antibody)

The antigen recognition site of the anti-adenovirus monoclonal antibody obtained in Example 1 was verified by Western blotting and LC-MS/MS.

### 1. Preparation of Concentrated-Adenovirus Liquid

Adenovirus was allowed to infect A549 cells, and cultured. On culture day 7, the adenovirus-infected cells were recovered and disrupted by ultrasonication. Cell residues were removed from the disrupted-cell liquid by centrifugation to obtain a concentrated-adenovirus liquid.

### 2. Preparation of Sample without Reduction Treatment

A two-fold dilution series of the concentrated-adenovirus liquid obtained in 1 was prepared, and supplemented with reagents having the respective final concentrations: 62.5 mM Tris-HCl (pH 6.5), 10 w/v% glycerol, 2.3 w/v% SDS, and 0.05% BPB (dye). The resulting mixture was subjected to a conventional method SDS-PAGE without being thermally denatured.

### 3. Preparation of Sample with Reduction Treatment

A two-fold dilution series of the concentrated-adenovirus liquid obtained in 1 was prepared, and supplemented with reagents having the respective final concentrations: 62.5 mM Tris-HCl (pH 6.5), 10 w/v% glycerol, 2.3 w/v% SDS, 0.05% BPB (dye), and 5% 2-mercaptoethanol. After being thermally denatured at 95°C for 1 minute, the resulting mixture was subjected to a conventional method SDS-PAGE.

### 4. Western Blotting

The gels electrophoresed in 2 and 3 were transferred to a PVDF membrane. After blocking of the membrane using skim milk, the membrane was sufficiently washed with PBS-Tween. The membrane was then allowed to react with the anti-adenovirus antibody whose concentration was adjusted to 3.8 µg/mL using PBS-Tween, at room temperature for 1 hour. After sufficiently washing the membrane with PBS-Tween, the membrane was allowed to react with a 3000-fold diluted HRP-labeled anti-mouse antibody at room temperature for 1 hour. After sufficiently washing the membrane with PBS-Tween, signals were detected using a chemiluminescence detection reagent.

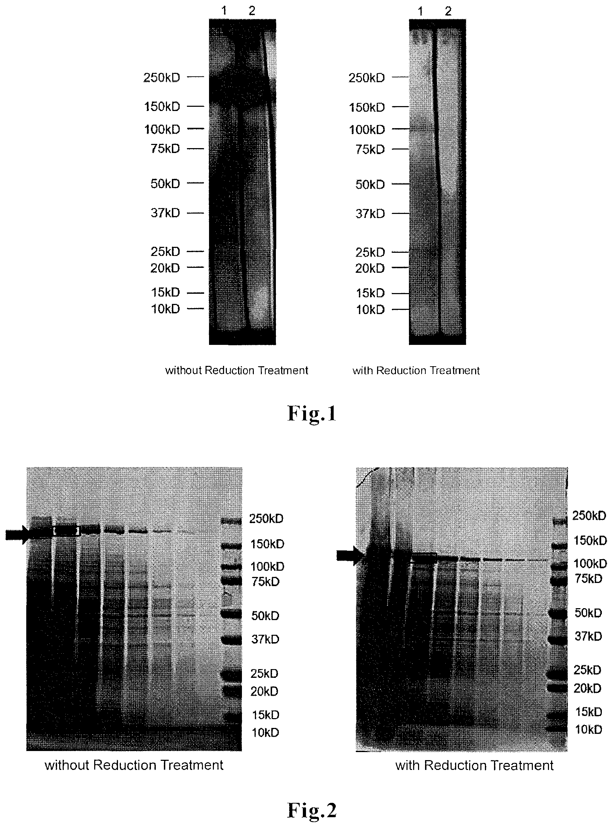

The results of Western Blotting are shown in Fig. 1.

A shown in Fig. 1, two antibodies (the antibody 1 and the antibody 2) produced in Example 1 reacted strongly with the approximately 200 kD protein (hexon trimer) contained in the sample obtained in 2 (without reduction treatment) (the left diagram). It is considered that the reduction treatment caused the hexon trimer to become a monomer, and it was recognized that the sample obtained in 3 (with reduction treatment) reacted very weakly with the approximately 100 kD protein (hexon monomer) (right diagram).

The results obtained by staining, with CBB, the gels electrophoresed in 2 and 3 are shown in Fig. 2.

As shown by the arrow in Fig. 2, the main protein contained in the sample obtained in 2 (without reduction treatment) was found at approximately 200 kD (the left diagram), and that contained in the sample obtained in 3 (with reduction treatment) was found at 100 to 150 kD (the right diagram). Each stained region denoted by a rectangle in the diagram was cut out, then hydrolyzed with trypsin, and analyzed by LC-MS/MS to give an amino acid sequence. The peptide fragment obtained was analyzed using Mascot (Ver. 2.5) (from Matrix Science Inc.) and Scaffold (from Proteome Software, Inc.), resulting in revealing that both of the stained regions contained a hexon protein of adenovirus as a main constituent.

Fig. 1 and Fig. 2 have revealed that both of the two antibodies obtained in Example 1 reacted more strongly with a protein of a hexon trimer than with a protein of a hexon monomer, and reacted weakly with a monomeric hexon protein.

## Claims

1. A monoclonal antibody or an antigen-binding fragment thereof, which undergoes antigen-antibody reaction with a hexon trimer of adenovirus.

2. The monoclonal antibody or the antigen-binding fragment thereof according to claim 1, wherein the monoclonal antibody undergoes antigen-antibody reaction more strongly with the hexon trimer than with a hexon monomer.

3. An immunoassay for adenovirus, comprising performing the immunoassay of adenovirus by using antigen-antibody reaction between the monoclonal antibody or the antigen-binding fragment thereof according to claim 1 or 2 and adenovirus in a sample.

4. The immunoassay according to claim 3, wherein the immunoassay is a sandwich method, and the monoclonal antibody or the antigen-binding fragment thereof is used as at least any one of a label or a solid phase.

5. An immunoassay device for adenovirus, comprising the monoclonal antibody or the antigen-binding fragment thereof according to claim 1 or 2.
